# EUROPEAN PATENT APPLICATION

(11) **EP 3 660 432 A1**
(43) Date of publication of application: **03.06.2020**
(21) Application number: 18851361.8
(22) Date of filing: 29.08.2018
(51) Int. Cl.: F25D 29/00, G06Q 30/06, G16H 50/30, G06Q 50/10

(54) **REFRIGERATOR**

(30) Priority: 30.08.2017 US 201762552068 P; 28.08.2018 KR 20180101151
(71) Applicant: Samsung Electronics Co., Ltd., Suwon-si, Gyeonggi-do 16677 (KR)
(72) Inventor: KOH, Justin, Suwon-si Gyeonggi-do 16677 (KR); DIVYA, Shah, Suwon-si Gyeonggi-do 16677 (KR); YUN, So Young, Suwon-si Gyeonggi-do 16677 (KR)
(74) Representative: Walaski, Jan Filip
(86) International application number: PCT/KR2018/010009
(87) International publication number: WO 2019/045456

(57) **Abstract**

Provided is a refrigerator, and more specifically, a refrigerator that provides an optimized screen for health care of a user on a front display thereof. The refrigerator includes: a storage compartment; a display configured to provide a screen for health care of a user; and a controller configured to control display of the display, wherein the screen for health care of the user includes at least one of health score graphics representing a score quantified from information regarding a health of the user, activity score graphics representing a score quantified from an activity of the user, meal planning graphics representing a meal planning of the user, or nutrition information graphics representing nutrition information of foods stored in the storage compartment, and further includes at least one icon of a health score management function icon, a step count management function icon, a meal planning management function icon, and a nutrition balance management function icon, of the use.

## Description

### [Technical Field]

The disclosure relates to a refrigerator, and more specifically, to a refrigerator configured to provide an optimized screen for health care of a user on a front display thereof.

### [Background Art]

A refrigerator is a device that keeps stored contents, such as foods, beverage, and the like, in a fresh state for a long time, and includes a storage compartment provided for freezing or refrigerating the stored contents, and a compressor, a condenser, an expansion valve, an evaporator, and the like that are provided in a machine room to perform a cooling cycle of compression-condensation-expansion-evaporation.

Such a refrigerator maintains the temperature in the storage compartment at a freezing temperature or refrigerating temperature using cold air that is heat-exchanged during the evaporation process of the cooling cycle.

Recently, refrigerators are in the trend of becoming larger in the capacity to store contents that are getting diverse and increasing in the quantity with improvement of standards of living, and are also providing more diverse functions for user convenience.

### [Disclosure]

### [Technical Problem]

The disclosure is directed to providing a refrigerator having a front display in which a screen optimized for health care of a user is provided. In detail, the disclosure is directed to providing a refrigerator in which at least one piece of information among a user's health score, a user's step count, a meal planning, or a nutrition balance is provided on a front display thereof.

### [Technical Solution]

One aspect of the disclosure provides a refrigerator including: a storage compartment; a display configured to provide a screen for health care of a user; and a controller configured to control display of the display, wherein the screen for health care of the user includes at least one of health score graphics representing a score quantified from information regarding a health of the user, activity score graphics representing a score quantified from an activity of the user, meal planning graphics representing a meal planning of the user, or nutrition information graphics representing nutrition information of foods stored in the storage compartment.

The screen for health care of the user may be provided in a form of a widget in one area of a home screen provided in the display.

The screen for health care of the user may provide at least one of the health score graphics, the activity information graphics, the meal planning image, or the nutrition information graphics according to a user's setting.

When a user's touch is input to the health score graphics, the display may display a health score management main screen including an area for providing an average health score of a plurality of users who are registered in advance and an area for providing respective individual scores of the plurality of users.

The area for providing the average health score of the plurality of users may include average health score information calculated on the basis of the respective individual scores of the plurality of users.

The area for providing the respective individual scores of the plurality of users may be provided with a user selection tab including at least one piece of information among an image, a name, a heart rate, sleeping hours, and a step count, of each of the plurality of users.

When a user's touch is input to the user selection tab, the display may display a health score management auxiliary screen including the at least one piece of information among the image, the name, the heart rate, the sleeping hours, or the step count, of a selected user.

When a user's touch is input to the activity score graphics, the display may display an activity score management main screen for visually providing step counts of a plurality of users who are registered in advance.

When a user's touch is input to the meal planning graphics, the display may display a meal planning management main screen including at least one of a day-of-week management area, a member management area, a recommended recipe providing area, or a recipe management area.

The day-of-week management area may be provided with a plurality of day icons, and when a user's touch is input to at least one day icon among the plurality of day icons, the display may display the recommended recipe providing area and the recipe management area of a day corresponding to the at least one day icon.

The member management area may be provided with respective images of a plurality of users who are registered in advance, and when a user's touch is input to at least one image among the respective images of the plurality of users, the display may display the recommended recipe providing area and the recipe management area of remaining users except for a user corresponding to the at least one image, to which the user's touch is input, among the plurality of users.

The refrigerator may further include an imager configured to collect food information of the storage compartment, wherein the controller may display a recommended recipe identified on the basis of the food information collected by the imager in the recommended recipe providing area.

The controller may determine the recommended recipe provided to the recommended recipe providing area on the basis of at least one of the food information collected by the image, a user's preference, and shelf life information of a food.

The recommended recipe providing area may be provided in a plurality of pages according to a number of recommended recipes determined by the controller.

The recipe management area may display a recipe selected by a user among recipes provided in the recommended recipe providing area, and include a shopping list button provided to enter a page for checking a shopping list on ingredients required for cooking recipes contained in the recipe management area.

When a user's touch is input to the nutrition information graphics, the display may display a nutrition balance management main screen including at least one of a nutrition display area including nutrition icons for visually providing nutrition information of foods provided in the storage compartment and a food recommendation area for recommending a food required for replenishing insufficient nutrition.

The nutrition display area may include nutrition icons that are classified according to a preset criterion and provide nutrition information of foods accommodated in the storage compartment.

The nutrition icon may be provided to visually provide percentile information of the amount of nutrient contained in the storage compartment on the basis of a preset recommended intake for a nutrient, which is criteria for classifying the nutrition icons.

The refrigerator may further include a communicator for receiving user's health information from an external device, wherein the controller may display the display to provide a screen for user's health care on the basis of the user's health information received from the communicator.

The external device may include at least one of a user terminal or a server provided to communicate with the user terminal.

### [Advantageous Effects]

With the refrigerator according to the disclosed embodiments, a screen optimized for health care of a user on a front display is provided, so that user convenience can be achieved. In detail, at least one piece of information among a user's health score, a user's step count, a meal planning, or nutrition balance is provided on a front display of the refrigerator, so that an effective health care tool can be provided.

### [Description of Drawings]

FIG. 1 is a perspective view illustrating a refrigerator according to an embodiment.
FIG. 2 is a perspective view illustrating the interior of the refrigerator shown in FIG. 1.
FIG. 3 is a view illustrating a refrigerator system including a refrigerator according to an embodiment.
FIGS. 4 and 5 are control block diagrams illustrating a refrigerator according to an embodiment.
FIG. 6 is a view illustrating an example of a home screen of a refrigerator in which a health care widget is provided.
FIGS. 7 to 9 are views for describing a health score management function.
FIGS. 10 and 11 are views for describing a step count management function of a user.
FIG. 12 is a view illustrating a meal planning management main screen.
FIG. 13 is a view illustrating a shopping list management screen.
FIG. 14 is a view for describing a main function of a meal planning management main screen.
FIG. 15 is a view for describing a main function of a shopping list screen.
FIGS. 16 to 18 are views for describing a nutrition balance management function.

### [Mode for the Invention]

Like numerals refer to like elements throughout the specification. Not all elements of embodiments of the present disclosure will be described, and description of what are commonly known in the art or what overlap each other in the embodiments will be omitted. The terms as used throughout the specification such as "- part", "∼ module", "∼ member", "∼ block", etc., may be implemented in software and/or hardware, and a plurality of "∼ parts", "∼ modules", "∼ members", or "∼ blocks" may be implemented in a single element, or a single "- part", "∼ module", "∼ member", or "∼ block" may include a plurality of elements.

It will be further understood that the term "connect" or its derivatives refer both to direct and indirect connection, and the indirect connection includes a connection over a wireless communication network.

It will be further understood that the terms "comprises" and/or "comprising," when used in this specification, specify the presence of stated features, integers, steps, operations, elements, and/or components, but do not preclude the presence or addition of one or more other features, integers, steps, operations, elements,

In the specification, it will be understood that, when a member is referred to as being "on/under" another member, it may be directly on/under the other member, or one or more intervening members may also be present.

The terms including ordinal numbers like "first" and "second" may be used to explain various components, but the components are not limited by the terms. The terms are only for the purpose of distinguishing a component from another.

As used herein, the singular forms "a," "an" and "the" are intended to include the plural forms as well, unless the context clearly indicates otherwise.

Reference numerals used for method steps are just used for convenience of explanation, but not to limit an order of the steps. Thus, unless the context clearly dictates otherwise, the written order may be practiced otherwise.

Hereinafter, the operating principles and embodiments of the disclosure will be described with reference to the accompanying drawings.

A refrigerator according to an embodiment may provide a home screen provided to effectively manage the health of registered users through a display provided on the front of the refrigerator. The home screen may include a health care screen or widget. Here, the health care screen may be provided in the form of a widget.

Terms used in the specification are defined as follows.

First, the health care widget is defined as a tool in which frequently used functions are collected such that registered users are effectively manage their health. The health care widget provides users with effective health care tools by providing the registered user with a health score management function, a step count management function, a meal planning management function, and a nutrition balance management function, of the user.

In addition, the user is a person who uses a refrigerator within a predetermined space while being registered in advance to provide the refrigerator with his/her health information. Hereinafter, for the sake of convenience in description of the disclosure, the concept of the user is defined as family members who use the refrigerator together in a predetermined space. However, the user is not limited to the family and it should be broadly understood as a concept that includes other units of users who use the refrigerator together in a certain space.

In addition, food is defined as a broad concept including a cooked food item contained in the refrigerator and ingredients required for cooking. As such, a food may refer to a concept including food ingredients.

Before describing embodiments of the disclosure in detail, the configuration of the refrigerator according to the embodiment will be described first for the sake of understanding of the disclosure.

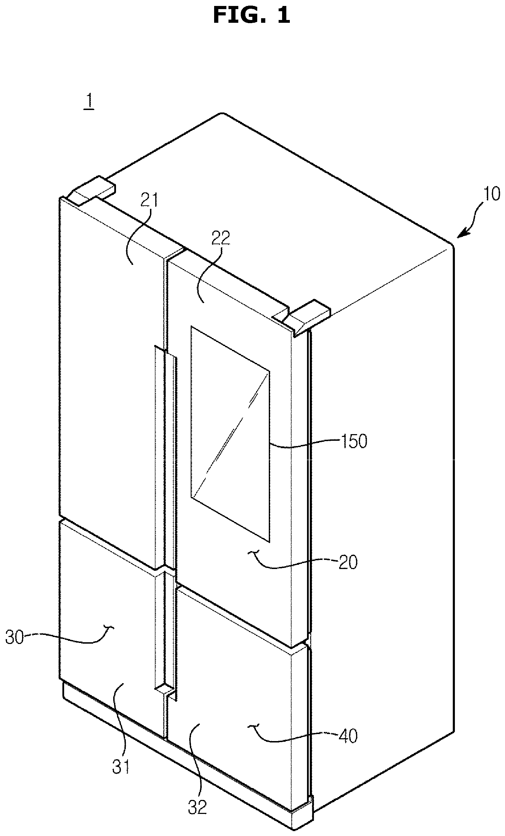

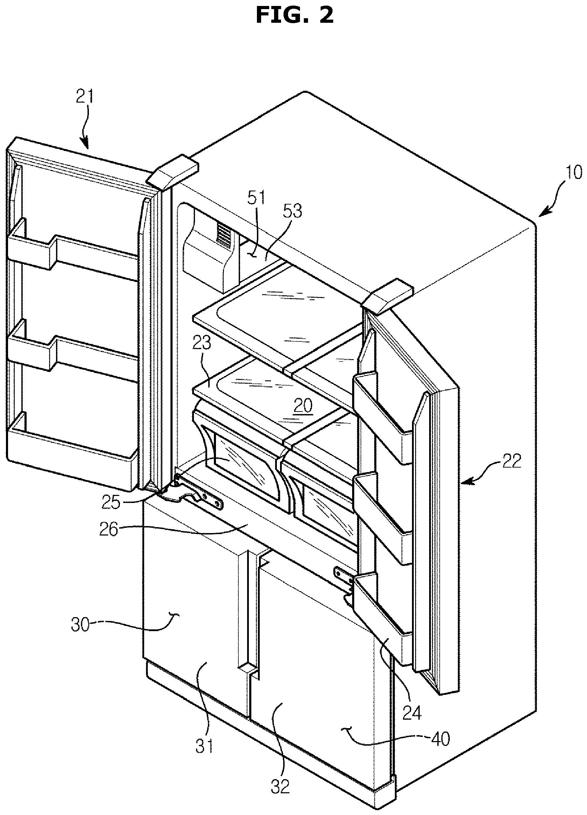

FIG. 1 is a perspective view illustrating a refrigerator according to an embodiment, and FIG. 2 is a perspective view illustrating the interior of the refrigerator shown in FIG. 1.

Referring to FIGS. 1 and 2, the refrigerator 1 according to the embodiment includes a body 10, storage compartments 20, 30, and 40 formed in the body 10, and a cool air supplying apparatus for supplying cool air to the storage compartments 20, 30, and 40.

The body 10 may include an inner case forming the storage compartments 20, 30, and 40, an outer case forming an external appearance by being coupled to an outer side of the inner case, and a heat insulation member disposed between the inner case and the outer case and configured to thermally insulate the storage compartments 20, 30, and 40.

The storage compartments 20, 30, and 40 may be partitioned into the storage compartment 20 on the upper side and the storage compartment 30 and 40 on the lower side by a first intermediate partition 26. In addition, the storage compartments 30 and 40 on the lower side may be partitioned into the storage compartment 30 on the left side and the storage compartment 40 on the right side with respect to the front surface of the refrigerator 1 by a second intermediate partition (not shown). For the sake of convenience in description, the following embodiments may assume that the storage compartment 20 on the upper side is provided as a refrigerating compartment 20, the storage compartment 30 on the lower left side is provided as a freezing compartment 30, and the storage compartment 40 on the lower right side is provided as a temperature changing compartment 40.

The refrigerating compartment 20 is maintained at a temperature of about 3 °C to keep foods refrigerated, the freezing compartment 30 is maintained at a temperature of about -18.5 °C to keep foods frozen, and the temperature changing compartment 40 changes in the temperature according to a user's preference to keep foods.

The refrigerating compartment 20 may be provided with a shelf 23 on which foods may be placed and at least one storage box 25 for hermetically storing foods.

In addition, on the upper corner of the refrigerating compartment 20, an ice making compartment 51 for generating ice may be formed to be partitioned from the refrigerating compartment 20 by an ice making compartment case 53.

In addition, the refrigerating compartment 20 may be provided with a water tank 60 capable of storing water. The water tank 60 may be provided between a plurality of the storage boxes 25 as shown in FIG. 2, but the water tank 60 is not limited thereto as long as water in the water tank 60 is cooled by cold air in the refrigerating compartment 20.

The refrigerating compartment 20 and the freezing compartment 30 each have a front side thereof open for foods to be inserted or withdrawn therethrough. The open front side of the refrigerating compartment 20 may be opened and closed by a pair of refrigerating compartment doors 21 and 22 hinged to the body 10. The open front sides of the freezing compartment 30 and the temperature changing compartment 40 may be opened and closed by a pair of doors 31 and 32 hinged to the body 10. The doors 21 and 22 of the refrigerating compartment 20 may be provided at the rear side thereof with a door guard 24 on which foods are stored.

On the other hand, the refrigerating compartment doors 21 and 22 may be provided at a rear side edge portion thereof with a gasket (not shown) for sealing between the refrigerating compartment doors 21 and 22 and the body 10 when the refrigerating compartment doors 21 and 22 are closed to hold the cold air of the refrigerating compartment 20. In addition, one of the refrigerating compartment doors 21 and 22 may be provided with a rotating bar (not shown) that seals between the refrigerating compartment doors 21 and 22 when the refrigerating compartment doors 21 and 22 are closed to hold the cold air of the refrigerating compartment 20.

In addition, the storage compartment doors 21, 22, 31, and 32 may be provided on the rear side edge portion thereof with a sensor 70 for detecting the opening and closing of the doors of the refrigerator 1. The sensor 70 may be provided by such configuration as a capacitive sensor, but the example of the sensor 70 is not limited thereto.

In addition, one of the refrigerating compartment doors 21 and 22 may be provided with a control panel 150 for checking the state of the refrigerator 1 from the outside or setting the operating conditions of the refrigerator 1 without opening the refrigerating compartment doors 21 and 22. The control panel 150 may be provided as a large display having a size of about 20 inches to realize convenience of user. However, the size of the control panel 150 is not limited to the numerical example described above.

A display 160 of the control panel 150 may provide a home screen for effectively managing the health of registered users. In detail, the display 160 may provide a home screen including a health care widget in which frequently used functions are collected such that registered users effectively manage their health.

The health care widget may provide "health score management function", "user's step count management function", "meal planning management function" and "nutrition balance management function" of the user. Each function will be described in detail as follows.

First, the "health score management function" of the user is a function to provide a health score by collecting pieces of health-related factor information of users (for example, family members) registered in advance and quantifying the collected pieces of information. Here, the health-related factor information refers to information for diagnosing the user's health, such as height, weight, heart rate, sleeping hours, and step count of the user. The refrigerator according to the disclosure may provide the display with the health scores of the previously registered users to provide the users with a comprehensive understanding of their lifestyle.

The "user's step count management function" is a function to provide step count ranking information together with step count information of registered users. Here, the pieces of step count information of the users may be individually provided for respective users, and according to embodiments, the total step count information of the registered users may be provided.

The "meal planning management function" is a function to provide the user with a recipe on the basis of ingredient information of ingredients contained in the refrigerator. The disclosure provides recipes to users, e.g., family members, on the basis of pieces of information, such as ingredients in the refrigerator, user preferences, health priority, shelf life of ingredients, and the like, so that users may be provided with recipes without checking foods in the refrigerator one by one.

The "nutrition balance management function" is a function to analyze foods in the refrigerator and provide recommendations for nutrition items and ingredients for healthy eating habit. The "nutrition balance management function" according to the disclosure visually informs whether recommendations for preset food groups are satisfied on the basis of the user's preference, health state, and health goal. Here, the recommendations for each food group may be provided on the basis of the United States Department of Agriculture (USDA) recommendations.

The health care widget may basically provide the above described four functions, but the number and type of the health care functions provided by the health care widget may vary according to a user's setting.

The home screen may be provided with the health care width and various types of widget screens including an internal temperature management widget, a recipe recommendation widget, a shopping management widget, a note management widget, a time management widget, and a weather management widget.

In addition, the home screen may be provided with application icons provided by the refrigerator 1. Such widgets and application icons may be changed in position or size according to a user's setting, so that user convenience is realized.

Meanwhile, the refrigerator 1 according to the disclosure may be provided in various forms in addition to the forms shown in FIGS. 1 and 2. In other words, the refrigerator 1 according to the disclosure may be a concept including all types of refrigerators 1 capable of providing display on one surface of a body or a door. For the sake of convenience in description, the embodiment assumes the refrigerator 1 shown in FIGS. 1 and 2 .

The refrigerator 1 according to the disclosure may provide the health care widget described above on the assumption of the technical configuration which will be described below. Hereinafter, a technical configuration of the refrigerator 1 for providing various functions of the above-described health care widget will be described, and then the detailed functions of the health care widget provided through the display 150 of the refrigerator 1 according to the disclosure will be described in detail.

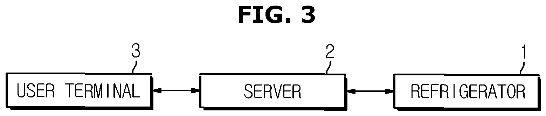

FIG. 3 is a view illustrating a refrigerator system including a refrigerator 1 according to an embodiment.

Referring to FIG. 3, the refrigerator system according to the embodiment includes a user terminal 3, a server 2, and a refrigerator 1.

The user terminal 3 is configured to collect health information of the user and transmit the collected health information to the server 2 or the refrigerator 1. Hereinafter, the health information of the user according to the disclosure refers to user's health-related information required to provide a health care screen (or widget) on the display of the refrigerator 1, and may conceptually include user's health-related information that may be collectable from the user terminal 30, for example, information regarding the user's height, weight, heat rate, sleeping hours, and step count.

The user terminal 3 may be implemented as a computer or a portable terminal capable of connecting to the server 2 or the refrigerator 1 through a network. Here, the computer may include, for example, a notebook computer, a desktop computer, a laptop PC, a tablet PC, a slate PC, and the like, each of which is equipped with a WEB Browser. The portable terminal is a wireless communication device having portability and mobility, including: all types of handheld based wireless communication devices, such as a personal communication system (PCS), a global system for mobile communications (GSM), a personal digital cellular (PDC), a personal handy phone system (PHS), a personal digital assistant (PDA), an international mobile telecommunication (IMT)-2000, a code division multiple access (CDMA)-2000, a w-code division multiple access (W-CDMA), a wireless broadband internet (WiBro) terminal, a smart Phone, and the like; and wearable devices, such as a watch, a ring, a bracelet, an ankle bracelet, a necklace, glasses, a contact lens, or a head-mounted-device (HMD).

The server 2 receives information collected by the plurality of user terminals 3 from the plurality of user terminals 3, processes the received information as required, and transmits the processed information to the refrigerator 1. The server 2 may exchange information with the plurality of user terminals 3 in a wireless communication method. The server 2 may serve as an authentication server for authenticating the user terminal 3, and thus may serve to mediate the user terminals 3 previously authenticated with respect to the specific refrigerator 1.

The refrigerator 1 collects information collected from the user terminal 3 through the server 2, and provides the collected information through a display provided on the front surface of the refrigerator 1. The refrigerator 1 may exchange information with the server 2 in a wireless communication method. The refrigerator 1 may provide a home screen including a health care widget through the display provided on the front side of the refrigerator 1. Hereinafter, the configuration of the refrigerator 1 will be described in detail with reference to the accompanying drawings.

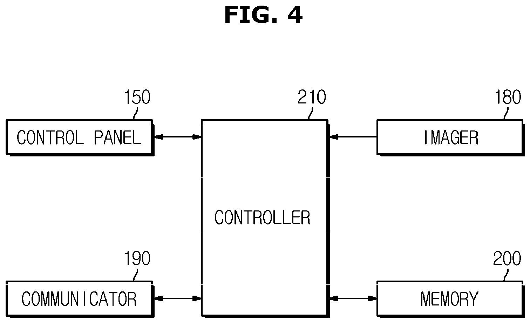

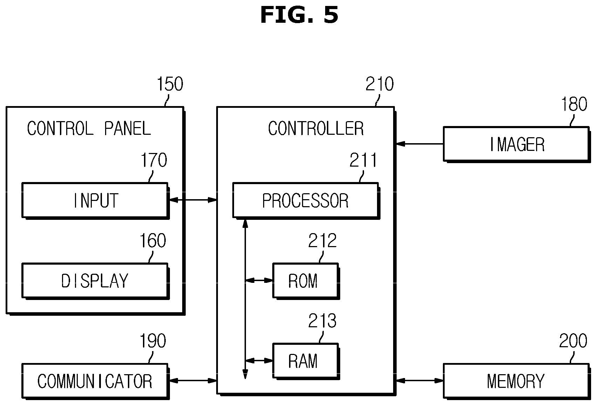

FIGS. 4 and 5 are control block diagrams illustrating a refrigerator 1 according to an embodiment.

Referring to FIGS. 4 and 5, the refrigerator 1 according to the embodiment includes a control panel 150, an imager 180, a communicator 190, a memory 200, and a controller 210.

The control panel 150 is provided for interface with the user, and may be arranged on the front side of the door of the refrigerator 1 for ease of access and manipulation of the user.

The control panel 150 may include an input 170 that receives a control command of a user, and a display 160 that visually provides information to the user.

The Input 170 may receive a control command for the refrigerator 1 from the user. The input 170 may be provided using a hard key method, a proximity sensor method, or a graphic user interface (GUI) method implemented through a touch pad for user input. When the input 170 is provided using a GUI method, such as using a touch pad, the input 170 may be implemented as a touch screen panel to form a mutual layer structure with the display. For the sake of convenience in description, the embodiment assumes that the input 170 is implemented as a touch screen panel.

The display 160 is provided to visually provide information to the user. The display 160 may provide a home screen including a health care screen. The health care screen may be provided in the form of a health care widget as described above. Accordingly, the user may share their lifestyle through the display of the refrigerator 1 using a health care widget as required, and according to embodiments, may receive recommended recipes or manage nutrition balance. Hereinafter, a method of providing the corresponding functions, together with examples of the screen for providing the corresponding functions, will be described in detail.

The display 160 may include a cathode ray tube (CRT) panel, a digital light processing (DLP) panel, a plasma display panel, a liquid crystal display (LCD) panel, an electro luminescence (EL) panel, an electrophoretic display (EPD) panel, an electrochromic display (ECD) panel, a light emitting diode (LED) panel, or an organic light emitting diode (OLED), but the type of the display 160 is not limited thereto.

The imager 180 is provided to collect food information in the refrigerator. Image information collected by the imager 180 is transmitted to the controller 210 and is used in the process of collecting food information in the refrigerator.

The imager 180 may include a plurality of image sensors, for example, a camera, and according to embodiments may also include a light source for providing light required for collecting image information of foods.

The imager 180 may be installed in each of the plurality of storage compartments. In addition, the imager 180 may be installed in a French bar of the storage compartment door, and may be installed corresponding in number to the number of shelves provided in the storage compartment.

A circuit, a lens, a mechanism or software of the camera included in the imager 180 may be provided such that the camera has an angle of view greater than or equal to a predetermined angle. In addition, the camera of the imager 180 may include a heater around the camera lens or the circuit. The heater may be controlled such that the temperature in the vicinity of the camera is maintained at a level higher than or equal to a certain temperature, to minimize an influence of condensation or moisture inside the refrigerating compartment on the lens or circuit of the camera.

The communicator 190 connects the refrigerator 1 to an external device under the control of the controller 210. The communicator 190 may allow health information of the user to be received from the server 2 under the control of the controller 210 such that the health information of the registered users is displayed through the display of the refrigerator 1.

The communicator 190 may include one of a wired Ethernet unit, a wireless LAN unit, and a short-range communication unit according to the performance and structure of the refrigerator 1.

The wireless LAN unit may be wirelessly connected to an access point (AP) at a site in which the AP is installed under the control of the controller 210. The wireless LAN unit may support a wireless LAN standard (IEEE1002.11x) of the Institute of Electrical and Electronics Engineers (IEEE). The short-range communication of the short-range communication unit may include Bluetooth, Bluetooth low energy, infrared data association (IrDA), Wi-Fi, Ultra-Wideband (UWB), Near Field Communication (NFC), and the like.

The memory 200 may store various types of data, control programs or applications for driving and controlling the refrigerator 1. In more detail, the memory 200 may store data, control programs, and applications for providing a health care widget to a user through the display of the refrigerator 1. According to an example, the memory 200 may store an application related to the health score function of the refrigerator 1, an application related to the user's step count management function, an application related to the meal planning management function, and an application related to the nutrition balance management function. Such an application may be an application initially stored or an application downloaded by a user while using the refrigerator 1.

The memory 200 may store a user interface (UI) associated with a control program and an application for providing a health care widget, an object (for example, an image, text, an icon, a button, and the like) for providing the UI, user registration information, documents, database, or related data. For example, the memory 200 may store unique identification (ID) and image information of users for identifying registered users, text and image information for visually providing health information of users received from the user terminal 3, and food image information of a food for identifying the food imaged by the imager 180, information for identifying a barcode attached to a package container of a food, image and recipe information for recommending a recipe, and the like.

The memory 200 may include at least one of a flash memory type memory, a hard disk type memory, a multimedia card micro type memory, a card type memory (e.g., a secure digital (SD) or an extreme digital (XD) memory, and the like), a random access memory (RAM), a static random access memory (SRAM), a read-only memory (ROM), an electrically erasable programmable read-only Memory (EEPROM), a programmable read-only memory (PROM), a magnetic memory, a magnetic disk, or an optical disk. However, the memory is not limited thereto and may be implemented in various forms generally known to those skilled in the art.

The controller 210 controls the overall operation of the refrigerator 1 and signal flow between the internal components of the refrigerator 1, and processes data. The controller 210 may execute a health care application or program stored in the memory 200 when a control command is input from a user or a preset condition is satisfied.

The controller 210 may include a processor 211, a ROM 212 configured to store a control program or application for controlling the refrigerator 1, a RAM 213 configured to store signals or data input from the outside of the refrigerator 1 or used as a storage corresponding to various tasks performed by the refrigerator 1. In the following description, the ROM and the RAM of the controller 210 may conceptually include the ROM and RAM of the memory 200.

The processor may include a graphic processor for graphic processing of images and videos. The processor may be implemented in the form of a system on chip (SoC) including a core and a graphic processing unit (GPU). According to embodiments, the controller 210 may include a graphic processing board including a graphic processor, a RAM, or a ROM on a separate circuit board electrically connected to the controller 210.

Hereinafter, the function of the health care widget provided in the display of the refrigerator 1 according to the disclosure will be described in detail with reference to the accompanying drawings.

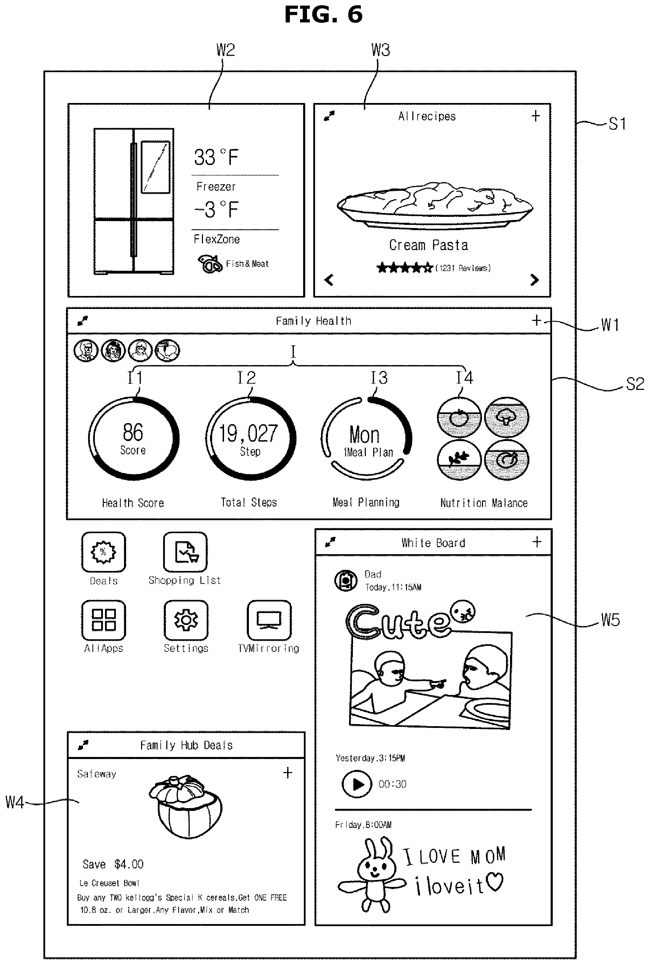

FIG. 6 is a view illustrating an example of a home screen S1 of a refrigerator 1 in which a health care widget W1 is provided.

Referring to FIG. 6, the home screen S1 of the refrigerator 1 according to the embodiment includes the health care widget W1. The health care widget W1 may be provided in the center of the home screen S1. In addition to the health care widget W1, the home screen S1 may be provided with various types of widgets, including an internal temperature management widget W2, a recipe recommendation widget W3, a shopping management widget W4, and a memo management widget W5. In addition, the home screen S1 may be provided with application icons provided by the refrigerator 1. Such widgets and application icons may be changed in position or size according to a user's setting so that the user's convenience is realized.

At the top of a main screen S2 of the health care widget W1, a text for describing the health care widget W1 may be provided, and at a lower side of the text, images of users registered in the health care widget W1 may be provided.

In the main screen S2 of the health care widget W1,icons I (I1, I2, I3, and 14) corresponding to functions provided by the health care widget W1 may be displayed. The icon I includes images and texts for describing the corresponding functions. The images and texts of the icon I visually provide health information provided by the functions. For example, the icon I may include a graph and a text for describing the graph. Accordingly, the graph and the text of the icon I may be updated in real time as the health information provided by the corresponding functions is updated.

Although the icons I (I1, 12, 13, and 14) for representative the representative functions provided by the health care widget W1 are illustrated as being displayed side by side in FIG. 6, the arrangement, shape, color, and the like of the icons I(I1, 12, 13, and 14) are not limited to the example shown in FIG. 6, and may vary according to a user's setting.

The number of icons may also vary according to a user's setting. In other words, icons frequently used in the health care widget W1 may be arranged according to a user's setting. For example, when a user sets only two frequently used functions in the healthcare widget W1, two icons may be displayed in the main screen S2 of the healthcare widget W1, in which case the two icons may be displayed to be larger than other icons shown in FIG. 6, and a text provided together with the two icons may be provided in more detail. According to embodiments, the user may set functions frequently used in the health care widget W1 to three or five or more.

Next, detailed functions that may be provided to the health care widget W1, that is, the health score management function, the user's step count management function, the meal planning management function, and the nutrition balance management function will be described. For the sake of convenience in description, an icon for the health score management function is referred to as a score icon I1, an icon for the user's step count management function is referred to as a step count icon 12, an icon for the meal planning management function is referred to as a meal planning icon 13, and an icon for the nutrition balance management function is referred to as a nutrition management icon 14.

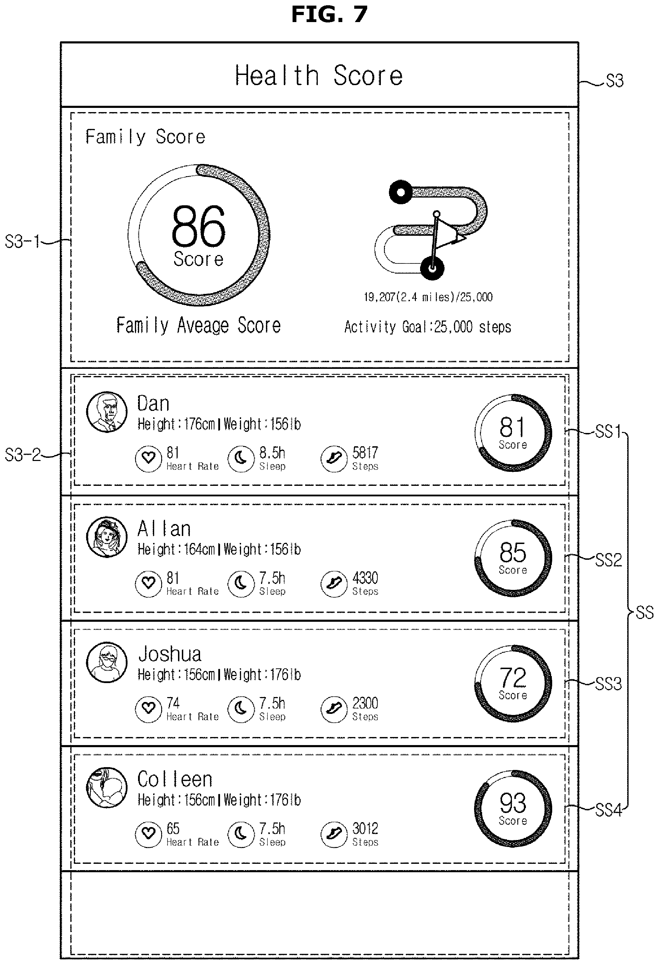

FIGS. 7 to 9 are views for describing the health score management function.

Referring to FIG. 7, a health score management main screen S3 is divided into an area S3-1 providing the average health score of family members and an area S3-2 providing the score of each of the family members. Hereinafter, the average health score of family members is referred to as a family score, and the score of each family member is referred to as an individual score.

The area S3-1 providing the family score may include a graph and a text for visually providing the family score. The graph may be provided in a circular form, but according to embodiments, may be provided in a bar graph form.

The family score may be calculated by arithmetic average or weighted average of the individual scores. A method of calculating the individual score will be described below.

The area S3-1 providing a family score may include the family score together with percentile information of the family score obtained by considering family scores of all the families in a specific region, location, and zip code in an ascending order.

The area S3-2 providing individual scores includes score providing tabs SS (SS1, SS2, SS3, and SS4) including texts and graphs visually providing individual scores together with detailed health information of family members. The score providing tabs SS may be provided corresponding in number to the number of registered family members.

For example, referring to FIG. 7, a score providing tab SS1 of a specific family member may provide information regarding the image, name, heart rate, sleeping hours, and step count of the user, and may provide individual score information calculated on the basis of the heart rate, sleeping hours, step count, and the like.

The individual score may be calculated by arithmetic average or weighted average of at least one of an activity score, a sleep score, or a nutritional score.

The activity score may be calculated as a percentage of an actual activity amount to the target activity amount of family members. Here, the target activity amount of family members may be calculated according to the target for activity amount, the height, the weight, the age, and the gender of each of the family members. The sleep score may be calculated as a percentage of an actual sleep amount to the target sleep amount. The nutrition score may be calculated on the basis of foods consumed by the family members, the actual activity amount, and the actual sleep amount.

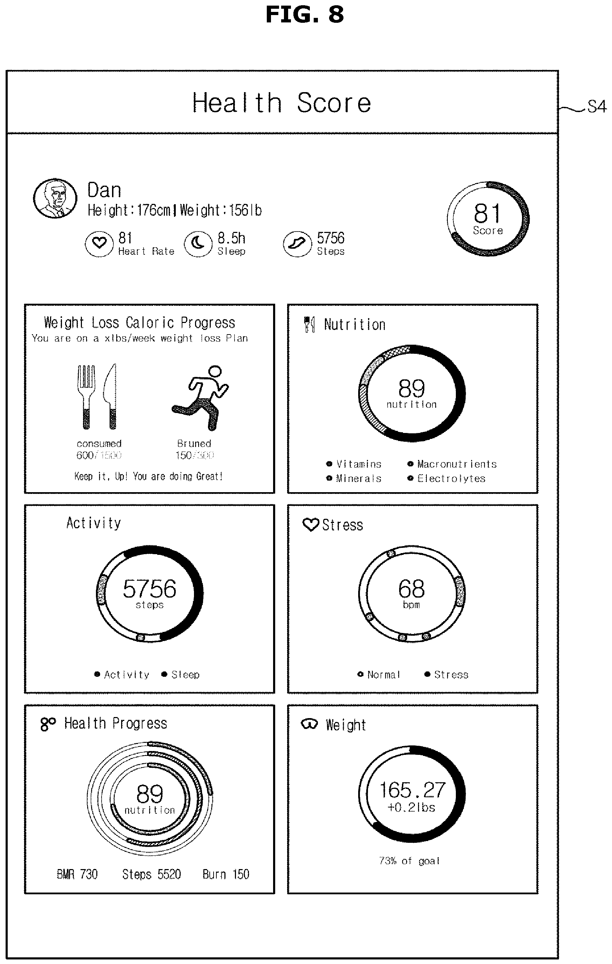

The health score management function may provide a health score management auxiliary screen for a specific family member, as shown in FIG. 8.

Referring to FIG. 8, a health score management auxiliary screen S4 provides a score providing screen of a specific family member and provides detailed health information of the specific family member. For example, the health score management auxiliary screen S4 may provide pieces of information regarding calorie burning, weight change, nutrient intake, activity amount information (step count, sleeping hours), heart rate, and the like in the form of a graph, a text, and a combination of a graph and a text.

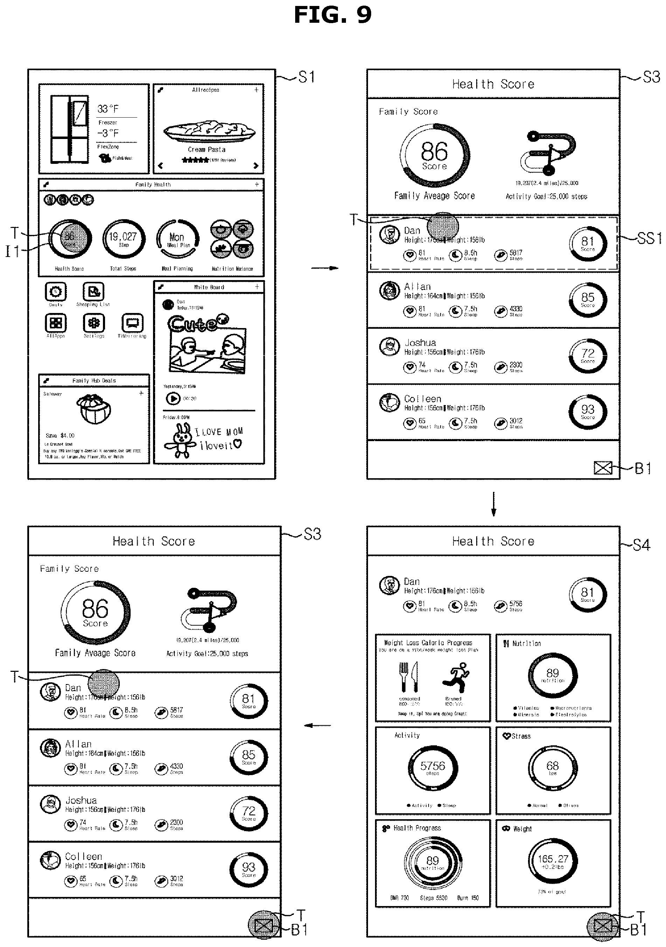

Referring to FIG. 9, when a user's touch T is input to the score icon I1 of the health care widget W1, the home screen S1 is switched to the health score management main screen S3 described with reference to FIG. 7. Subsequently, when a user's touch T is input to the score providing tab SS1 provided on the health score management main screen S3, the health score management main screen S3 is switched to the health score management auxiliary screen S4. When a touch T is input to a back button B1 of the health score management auxiliary screen S4 while the health score management auxiliary screen S4 is displayed in the display 160, the health score management auxiliary screen S4 is switched back to the health score management main screen S3, and when a touch T is input to a back button B1 of the health score management main screen S3, the health score management main screen S3 is switched to the home screen S1. According to embodiments, the back button B1 may be provided at any position on each of the screens S3 and S4, and may be provided on the control panel 150 to be physically or touchable.

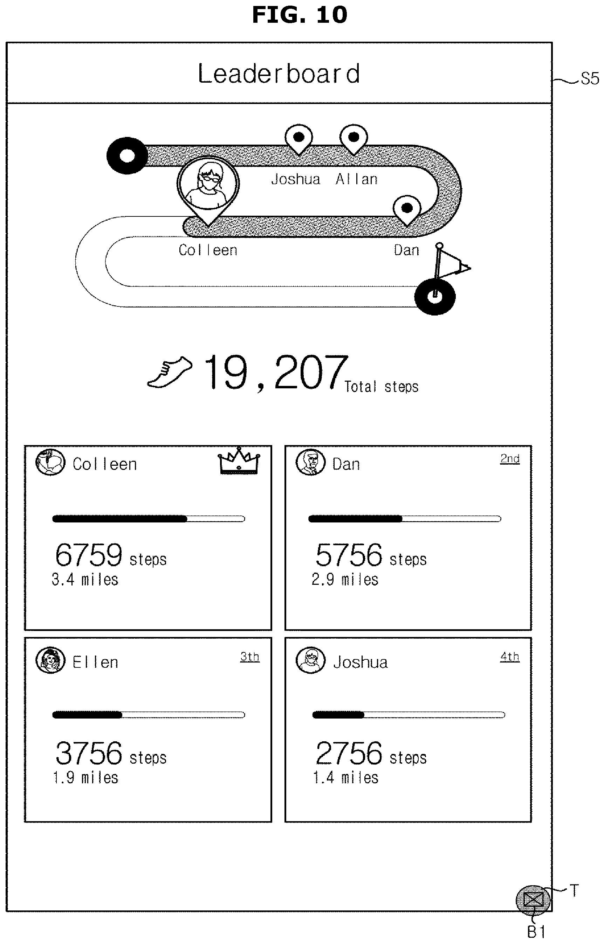

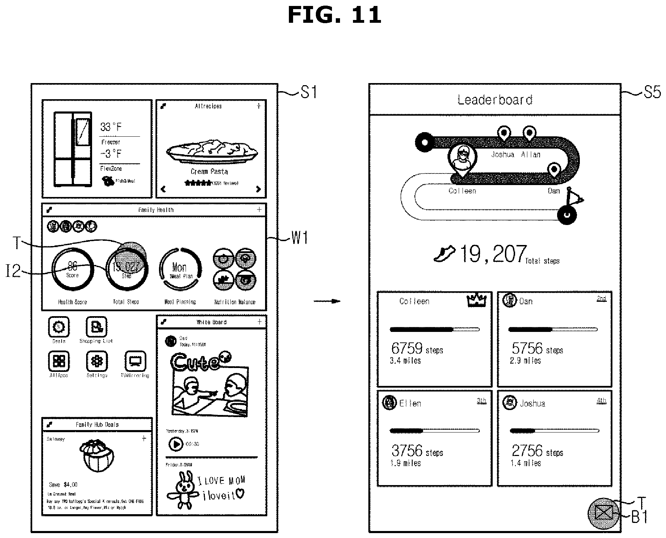

FIGS. 10 and 11 are views for describing a step count management function of a user.

Referring to FIG. 10, a user step count management main screen S5 includes graphs for visually providing the step counts of the family members, and a text for visually providing the total step count of the family members. The graph may include a main graph that comprehensively provides the step counts of the family members and an auxiliary graph that provides the step count of each family member. The main graph may be provided with the target step count, and markers for visually providing step counts achieved by family members compared to the target step count. The auxiliary graphs are provided for respective family members, and the arrangement of the respective auxiliary graphs may be determined according to the rankings of the step counts of the family members. In addition, a marker may be provided around the auxiliary graph of a member who has the highest step count to inform that the member has the highest step count.

Referring to FIG. 11, when a user's touch T is input to the step icon 12 of the health care widget W1, the home screen S1 is switched to the step count management main screen S5 described with reference to FIG. 10. Subsequently, when a user's touch T is input to a back button B1 provided at the bottom of the step count management main screen S5, the step count management main screen S5 is switched back to the home screen S1. According to embodiments, the back button B1 may be provided at any position on the step count management main screen S5, and may be provided on the control panel 150 to be physically or touchable.

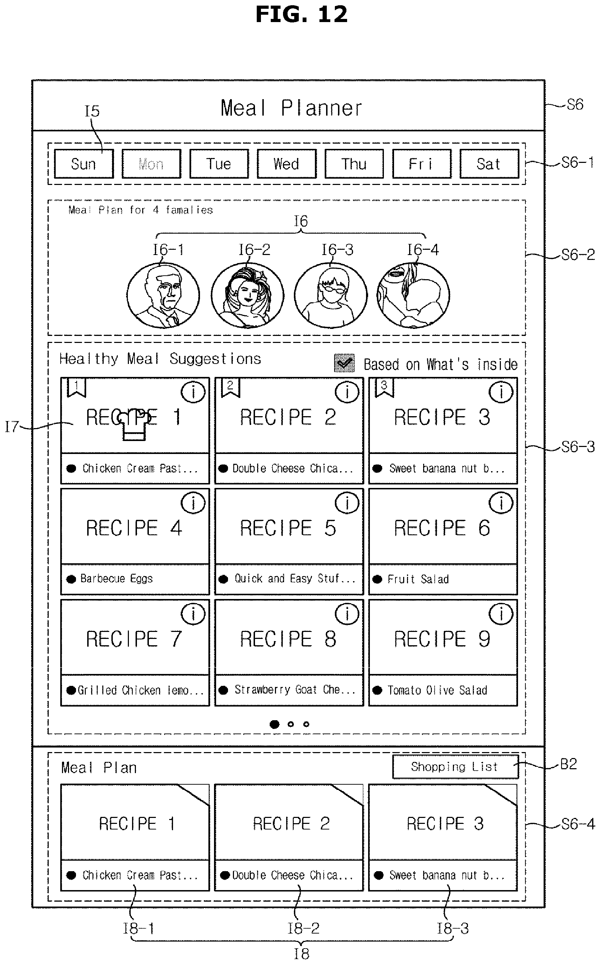

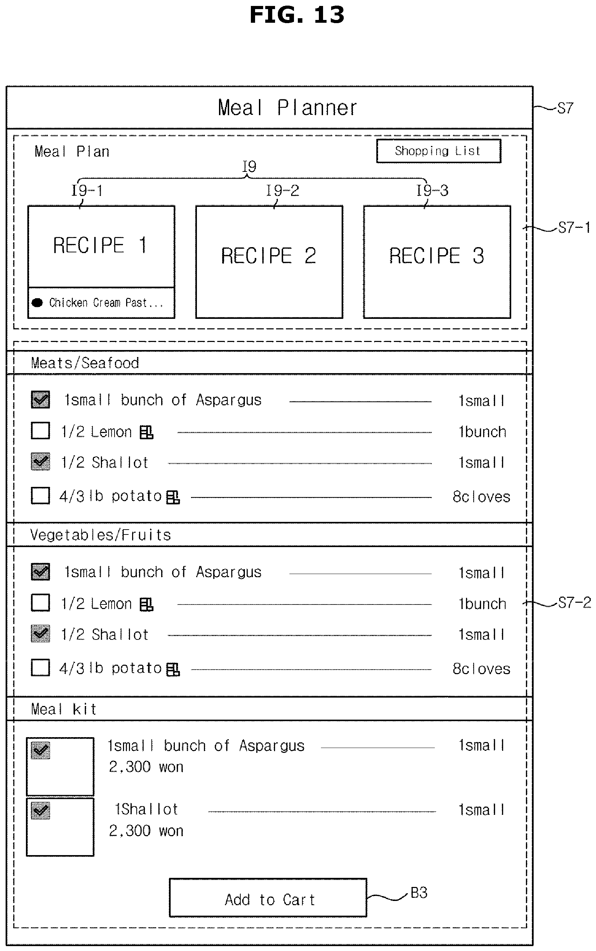

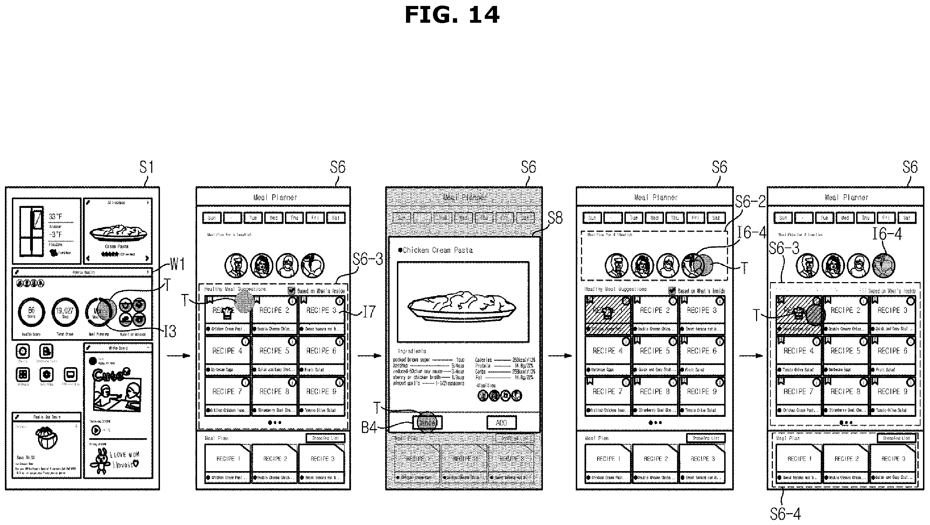

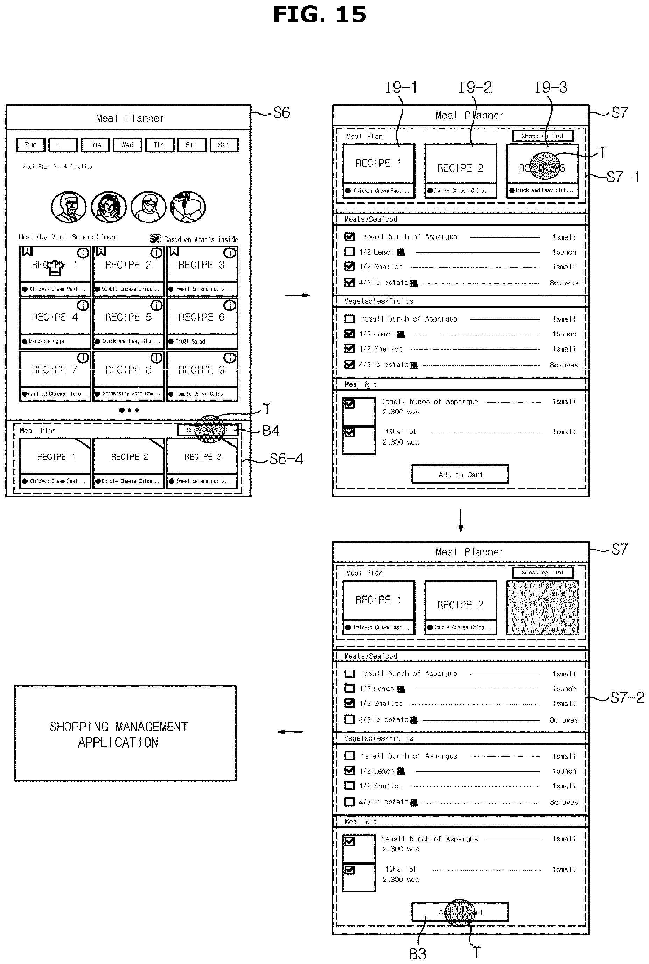

FIGS. 12 to 15 are views illustrating a meal planning management function. FIG. 12 is a view illustrating a meal planning management main screen, FIG. 13 is a view illustrating a shopping list management screen. FIG. 14 is a view for describing a main function of a meal planning management main screen, and FIG. 15 is a view for describing a main function of a shopping list screen.

Referring to FIG. 12, a meal planning management main screen S6 is divided into a day-of-week management area S6-1, a member management area S6-2, a recommended recipe providing area S6-3, and a recipe management area S6-4.

First, the day-of-week management area S6-1 is provided at the top of a meal planning management main screen S6. The user may select a day icon 15 provided in the day-of-week management area S6-1 to set a meal planning for the corresponding day. When a user's touch is input to one of the plurality of day icons 15, the member management area S6-2 of the meal planning management main screen S6 is fixed, and the recommended recipe providing area S6-3 and the recipe management area S6-4 corresponding to the day icon 15 are provided. On the other hand, when the members of the member management area S6-2 are differently set according to the day of weeks, the member management area S6-2 may be provided differently according to the day of weeks.

The member management area S6-2 may be provided at the lower side of the day-of-week management area S6-1. The member management area S6-2 is an area provided to select family members to be considered when planning a diet.

In the member management area S6-2, image icons 16 (16-1, 16-2, 16-3, and 16-4) of the family members are displayed. The meal planning management function is a function to manage the meal planning of the family members on the basis of the preferences of the family members together with the foods contained in the refrigerator, and the user may select the family members to be considered in the meal planning by touching the image icons of the family members. For example, the user may touch the image icon of a family member to switch the image icon of the family member to be in an active state (so as to be considered in the meal planning) or inactive (so as not to be considered in the meal planning). In this case, the recommended recipes provided in the recommended recipe providing area S6-3 to be described below are changed in real time according to the change of the family members.

The recommended recipe providing area S6-3 is provided at a lower side of the member management area S6-2. In the recommended recipe providing area S6-3, recommended recipe icons 17 for recommended recipes determined by an algorithm of the program stored in the controller 210 are provided. The recommended recipe providing area S6-3 may be provided in a plurality of pages according to the number of recommended recipes. The pages of the recommended recipe providing area S6-3 are switched when a drag touch is input to the recommended recipe providing area S6-3 in a left side and right side direction or in an upper side and lower side direction. Meanwhile, an icon for visually providing the current recipe providing page may be displayed in one area of the recommended recipe providing area S6-3.

The recommended recipes provided in the recommended recipe providing area S6-3 are prioritized and provided on the basis of the following factors.

First, the recommended recipe is determined on the basis of the food existing in the refrigerator. In more detail, when image information of the food collected by the camera provided in the refrigerator is transmitted to the controller 210, the controller 210 determines the type and amount of the food on the basis of the collected image information, and determines the type and priority of a recommended recipe on the basis of the determined type and amount of the food.

In addition, the recommended recipe is determined on the basis of preferences of the family members. In other words, the family members may perform pre-setting such that certain ingredients are excluded when providing recipes, and the type and priority of recommended recipes may be determined on the basis of the preferences of the family members.

In addition, the recommended recipe may be determined on the basis of the shelf life of foods existing in the refrigerator. In more detail, when an image of the food or bar code information marked on a container of the food collected through the camera provided in the refrigerator is transmitted to the controller 210, the controller 210 may determine the shelf life of the food on the basis of the collected image information, and determine the type and priority of a recommended recipe on the basis of the determined shelf life of the food.

The recommended recipes may be determined in type and priority on the basis of the above described factors, and the type and priority may be determined by reflecting a weight value for a specific factor.

At the lower side of the recommended recipe providing area S6-3, the recipe management area S6-4 is provided. The recipe management area S6-4 is an area for displaying recipes selected by the user among the recipes provided in the recommended recipe providing area S6-3. In the recipe management area S6-4, recipe icons 18 (18-1,18-2, and 18-3) selected by the user are displayed. In the recipe management area S6-4, a plurality of recipes may be managed for each day of the week, and for the sake of convenience in description, FIG. 12 illustrates an example that three recipes are managed.

In one area of the recipe management area S6-4, a shopping list button B2 for entering a page (hereinafter, referred to as a shopping list management screen) to check a shopping list of ingredients required for cooking the recipe contained in the recipe management area S6-4 may be provided.

Referring to FIG. 13, the shopping list management screen S7 is divided into a recipe display area S7-1 and a food ingredient providing area S7-2.

The recipe display area S7-1 is provided at the top of the shopping list management screen S7. The recipe display area S7-1 displays recipe icons 19 (19-1,19-2, and 19-3) for recipes based on which the shopping list is generated.

The user may touch the recipe icons 19 (19-1, 19-2, and 19-3) to adjust recipes based on which the shopping list is generated. For example, the user may switch a recipe icon to be in an inactive state (so as not to be considered when generating a shopping list) or in an active state (so as to be considered when generating a shopping list) by touching the recipe icon.

The food ingredient providing area S7-2 provides a food ingredient list required for cooking recipes selected in the recipe management area S6-4. Ingredient items of the Ingredient list includes check boxes. Accordingly, the food ingredient list generated by the food ingredient items having a check box marked may serve as a shopping list.

As described above, the recommended recipes provided in the recipe management area (S6-4 in FIG. 12) are determined on the basis of food ingredients in the refrigerator. Accordingly, items of food ingredients that do not currently exist in the refrigerator among food ingredients required for cook the selected recipes are provided as food items for which shopping is required, that is, a shopping list. In other words, the shopping list displays all the food ingredient items required for cooking the selected recipes while a check box for a food ingredient, for which shopping is required, among the displayed food ingredient items marked.

At the bottom of the shopping list management screen S7,an add-to-cart button B3 is provided. When a user's touch is input to the add-to-cart button B3, the generated shopping list is transmitted to a shopping management application of the refrigerator 1.

Next, the meal planning management function will be described with reference to a flowchart described below.

Referring to FIG. 14, when a user's touch T is input to the meal planning icon 13 of the health care widget W1, the home screen S1 is switched to the meal planning management screen S6 described with reference to FIG. 12. Subsequently, when a user's touch T is input to a recipe icon 17 among the recipe icons provided in the recommended recipe providing area S6-3 of the meal planning management screen S6, the meal planning management screen S6 is switched to be in an inactive state, and a pop-up page S8 providing detailed information of the corresponding recipe is provided. When a user's touch is input to a cancel button B4 provided at the lower side of the pop-up page S8, the pop-up page S8 is canceled, and the meal planning management screen S6 is switched back to be in an active state.

Subsequently, when a user's touch is input to one image icon 16-4 among the image icons of the family members provided in the member management area S6-2, the image icon 16-4 of the corresponding family member is switched to be in an inactive state, and recipes determined on the basis of the remaining family members except for the family member corresponding to the image icon 16-4 are provided to the recommended recipe providing area S6-3.

In the recommended recipe providing area S6-3, a plurality of recommended recipes are provided. When a user's touch T is input to one of the plurality of recommended recipes, the corresponding recipe is added to the recipe management area S6-4.

Next, the shopping list management function will be described.

Referring to FIG. 15, when a user's touch is input to the shopping list button B4 provided in the recipe management area S6-4, the meal planning management main screen S6 is switched to the shopping list management screen S7. Subsequently, when a user's touch is input to one recipe icon 19-3 among the recipe icons provided in the recipe display area S7-1 of the shopping list management screen S7, the corresponding recipe icon 19-3 is switched to be in an inactive state, and a shopping list regenerated on the basis of the recipes corresponding to the recipe icons 19-1 and 19-2 except for the recipe corresponding to the recipe icon 19-3 is provided to the food ingredient providing area S7-2.

Subsequently, when a user's touch T is input to the add-to-cart button B3 at the lower side of the shopping list management screen S7, the generated shopping list is transmitted to the shopping management application of the refrigerator 1.

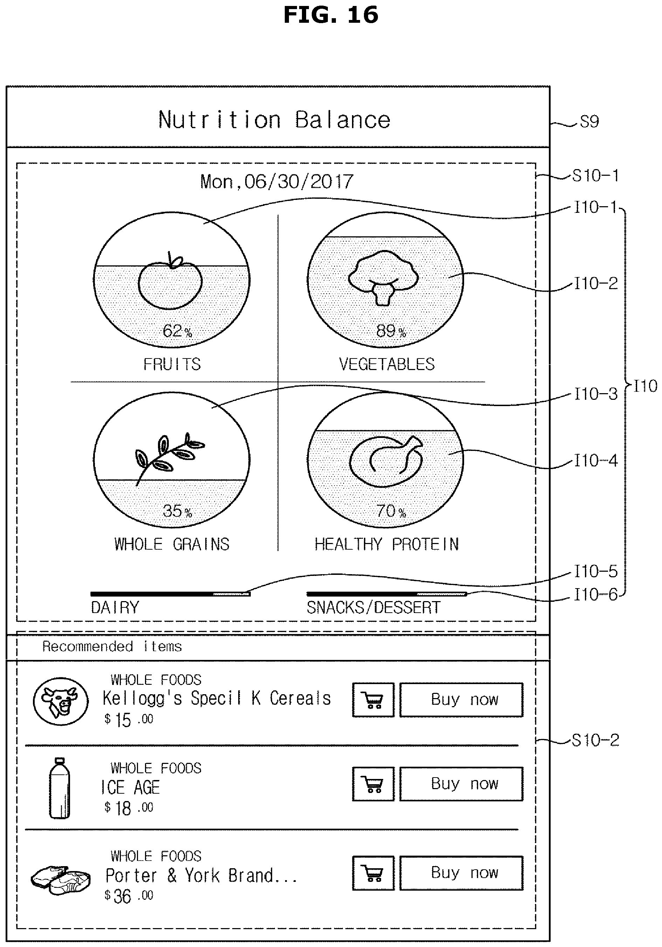

Next, FIGS. 16 to 18 are views for describing a nutrition balance management function.

Referring to FIG. 16, a nutrition balance management main screen S9 is divided into a nutrition display area S9-1 providing nutrition balance information, and a food recommendation area S9-2 recommending foods required to replenish insufficient nutrition.

In the nutrition display area S9-1, nutrition icons 110 (110-1, 110-2, 110-3, 110-4, 110-5, and 110-6) for visually providing nutrition information of foods provided in the refrigerator are provided. The nutrition icons 110 (110-1, 110-2, 110-3, 110-4, 110-5, and 110-6) may provide nutrition information of foods contained in the refrigerator in percentage units.

For example, among the nutrition icons 110 (110-1, 110-2, 110-3, 110-4, 110-5, and 110-6), some nutrition icons 110-1, 110-2, 110-3 and 110-4 may be classified according to a preset criterion, to provide nutrition information of foods contained in the refrigerator. In one example, the nutrition icons 110-1, 110-2, 110-3 and 110-4 may be classified by categories, such as "fruits", "vegetables", "whole grains" and "health proteins" However, the criterion for classifying the nutrition icons is not limited thereto, and various criteria capable of distinguishing nutrients such as by detailed units of nutrients or a unit grouping specific detailed units of nutrients may be used.

Some nutrition icons 110-5 and 110-6 among the nutrition icons 110 (110-1, 110-2, 110-3, 110-4, 110-5, and 110-6) may be arranged below the nutrition icons 110-1, 110-2, 110-3, and 110-4 in order of importance. Unlike to the nutrition icons 110-1, 110-2, 110-3 and 110-4, the nutrition icons 110-5 and 110-6 may be provided in the form of a bar, and may provide nutritional information of nutrients that have a lower importance.

The nutrition icon is provided to visually provide percentile information of the amount of nutrient currently contained in the refrigerator on the basis of a preset recommended intake for a nutrient, which is criteria for classifying the nutrition icons. Here, the recommended intake of nutrient may be provided on the basis of the united states department of agriculture (USDA) recommended food portion size. The recommended intake of nutrient may be provided on a daily intake basis, and according to embodiments, the recommended intake of nutrient may be provided on a weekly intake basis.

Users may easily generate a shopping list by identifying the recommended intake for a specific nutrient item on the basis of the percentile information displayed in the nutrition icon, and may have a balanced nutrient intake by visualizing the completion of nutrients for each food group when storing foods in the refrigerator.

The food recommendation area S9-2 provides a food recommendation list. The food recommendation list is provided in order of foods containing nutrients that are currently insufficient in balancing the nutrition of the foods held in the refrigerator. For example, FIG. 16 show lack of "whole grains" in comparison to other nutrients, and thus foods of a grain category are firstly recommended in the food recommendation list. A food recommendation algorithm in the food recommendation process according to the embodiment is determined by a pre-stored program, and the user's preference may be reflected in the food recommendation algorithm.

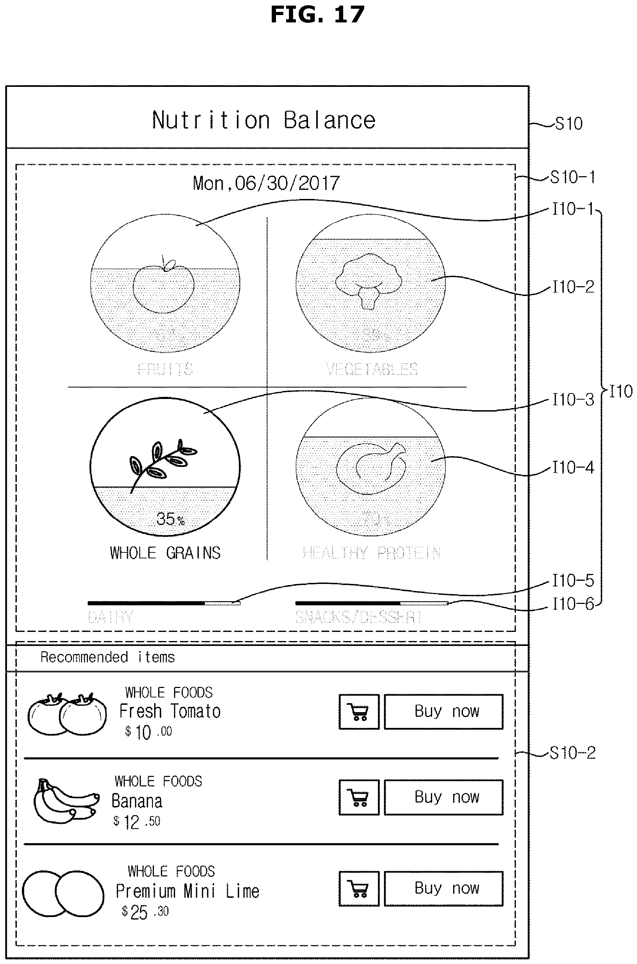

FIG. 17 is a nutrition balance management detailed screen S10, in which the nutrition balance management detailed screen S10 is divided into a nutrition display area S10-1 providing nutrition balance information for a specific food category, and a food recommendation area S10-2 recommending foods required for supplementing insufficient nutrition of the specific food category.

In the nutrition display area S10-1, a nutrition icon 110-1 visually providing nutrient information for a specific food category is provided. The nutrition balance management detailed screen S10 of FIG. 16 is a screen switched from the nutrition balance management main screen S9 of FIG. 15, and upon entering the nutrition balance management detailed screen S10, nutrient information for food categories except for a target food category desired to be provided in the nutrition balance management detailed screen S10 is switched to be in an inactivate state.

The food recommendation area S10-2 is provided with a food recommendation list for supplementing insufficient nutrients in the target food category. The food recommendation list is provided in the order of foods containing nutrients that are currently insufficient in balancing the nutrition of foods belonging to the target food category among the foods held in the refrigerator. A food recommendation algorithm of the food recommendation process according to the embodiment is determined by a pre-stored program, and a user's preference may be reflected in the food recommendation algorithm.

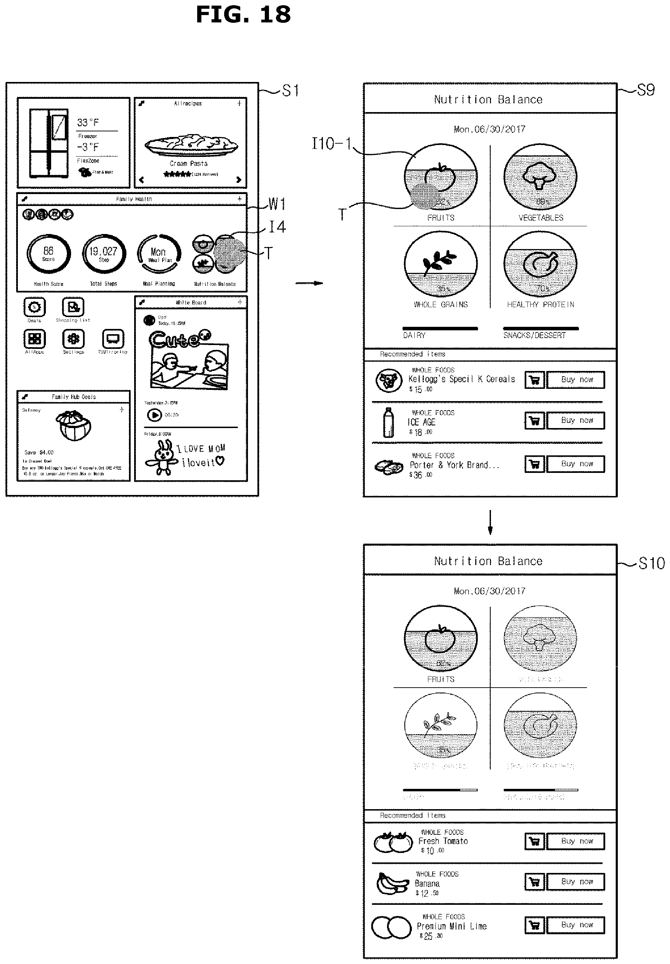

Referring to FIG. 18, when a user's touch T is input to the nutrition management icon 14 of the health care widget W1, the home screen S1 is switched to the nutrition balance management main screen S9 described with reference to FIG. 16. Subsequently, when a user's touch T is input to the nutrition icon 110-1 of the nutrition balance management main screen S9, the nutrition balance management main screen S9 is switched to the nutrition balance management detailed screen S1O for the food category of the nutrition icon 110-1.

Meanwhile, the disclosed embodiments may be embodied in the form of a recording medium storing instructions executable by a computer. The instructions may be stored in the form of program code and, when executed by a processor, may generate a program module to perform the operations of the disclosed embodiments. The recording medium may be embodied as a computer-readable recording medium.

The computer-readable recording medium includes all kinds of recording media in which instructions which may be decoded by a computer are stored, for example, a Read Only Memory (ROM), a Random-Access Memory (RAM), a magnetic tape, a magnetic disk, a flash memory, an optical data storage device, and the like.

Although exemplary embodiments of the disclosure have been described for illustrative purposes, those skilled in the art will appreciate that various modifications, additions and substitutions are possible, without departing from the scope and spirit of the disclosure. Therefore, exemplary embodiments of the disclosure have not been described for limiting purposes.

## Claims

1. A refrigerator comprising:
a storage compartment;
a display configured to provide a screen for health care of a user; and
a controller configured to control display of the display,
wherein the screen for health care of the user includes at least one of health score graphics representing a score quantified from information regarding a health of the user, activity score graphics representing a score quantified from an activity of the user, meal planning graphics representing a meal planning of the user, or nutrition information graphics representing nutrition information of foods stored in the storage compartment.

2. The refrigerator of claim 1, wherein the screen for health care of the user is provided in a form of a widget in one area of a home screen provided in the display.

3. The refrigerator of claim 1, wherein the screen for health care of the user provides at least one of the health score graphics, the activity information graphics, the meal planning image, or the nutrition information graphics according to a user's setting.

4. The refrigerator of claim 1, wherein when a user's touch is input to the health score graphics, the display displays a health score management main screen including an area for providing an average health score of a plurality of users who are registered in advance and an area for providing respective individual scores of the plurality of users.

5. The refrigerator of claim 4, wherein the area for providing the average health score of the plurality of users includes average health score information calculated on the basis of the respective individual scores of the plurality of users.

6. The refrigerator of claim 4, wherein the area for providing the respective individual scores of the plurality of users is provided with a user selection tab including at least one piece of information among an image, a name, a heart rate, sleeping hours, and a step count, of each of the plurality of users.

7. The refrigerator of claim 6, wherein when a user's touch is input to the user selection tab, the display displays a health score management auxiliary screen including the at least one piece of information among the image, the name, the heart rate, the sleeping hours, or the step count, of a selected user.

8. The refrigerator of claim 1, wherein when a user's touch is input to the activity score graphics, the display displays an activity score management main screen for visually providing step counts of a plurality of users who are registered in advance.

9. The refrigerator of claim 1, wherein when a user's touch is input to the meal planning graphics, the display displays a meal planning management main screen including at least one of a day-of-week management area, a member management area, a recommended recipe providing area, or a recipe management area.

10. The refrigerator of claim 9, wherein the day-of-week management area is provided with a plurality of day icons, and when a user's touch is input to at least one day icon among the plurality of day icons, the display displays the recommended recipe providing area and the recipe management area of a day corresponding to the at least one day icon.

11. The refrigerator of claim 9, wherein the member management area is provided with respective images of a plurality of users who are registered in advance, and when a user's touch is input to at least one image among the respective images of the plurality of users, the display displays the recommended recipe providing area and the recipe management area of remaining users except for a user corresponding to the at least one image, to which the user's touch is input, among the plurality of users.

12. The refrigerator of claim 9, further comprising an imager configured to collect food information of the storage compartment,
wherein the controller displays a recommended recipe identified on the basis of the food information collected by the imager in the recommended recipe providing area.

13. The refrigerator of claim 12, wherein the controller determines the recommended recipe provided to the recommended recipe providing area on the basis of at least one of the food information collected by the image, a user's preference, and shelf life information of a food.

14. The refrigerator of claim 11, wherein the recommended recipe providing area is provided in a plurality of pages according to a number of recommended recipes determined by the controller.

15. The refrigerator of claim 11, wherein the recipe management area displays a recipe selected by a user among recipes provided in the recommended recipe providing area, and includes a shopping list button provided to enter a page for checking a shopping list on ingredients required for cooking recipes contained in the recipe management area.
